# EUROPEAN PATENT SPECIFICATION

(11) **EP 3 878 959 B1**
(45) Date of publication and mention of the grant of the patent: **08.07.2026**
(21) Application number: 19881112.7
(22) Date of filing: 27.08.2019
(51) Int. Cl.: A01K 67/0276, A61K 48/00, A61K 49/00, A61P 1/16, C07K 14/78, C40B 30/06, G01N 33/50, G01N 33/68

(54) **USE OF ECM1 GENE-KNOCKOUT MOUSE IN SCREENING OF ANTI-HEPATIC FIBROSIS DRUG**
VERWENDUNG EINER ECM1-GENAUSSCHALTUNGSMAUS BEIM SCREENING VON WIRKSTOFFEN GEGEN LUNGENFIBROSE
UTILISATION D'UNE SOURIS KNOCK-OUT INACTIVANT LE GÈNE ECM1 DANS LE CRIBLAGE D'UN MÉDICAMENT ANTI-FIBROSE HÉPATIQUE

(30) Priority: 08.11.2018 CN 201811326908
(43) Date of publication of application: 15.09.2021
(73) Proprietor: Center for Excellence in Molecular Cell Science, Chinese Academy of Sciences, Xuhui District Shanghai 200031 (CN)
(72) Inventor: SUN, Bing, Shanghai 200031 (CN); FAN, Weiguo, Shanghai 200031 (CN); ZHANG, Yaguang, Shanghai 200031 (CN); LING, Zhiyang, Shanghai 200031 (CN)
(74) Representative: Mewburn Ellis LLP
(86) International application number: PCT/CN2019/102889
(87) International publication number: WO 2020/093760

(56) References cited:
- EP-A1- 3 913 002
- WO-A1-2020/088070
- CN-A- 102 552 910
- CN-A- 103 361 336
- CN-A- 107 801 691
- US-A1- 2014 273 275
- FAN WEIGUO: "190 ECM1, a Gate Keeper in Liver Restraining TGF-[beta] Activation to Maintain Homeostasis and to Prevent Fibrogenesis", HEPATOLOGY, vol. 68, 1 October 2018 (2018-10-01), pages 1 - 183, XP055940523, DOI: 10.1002/hep.30256
- JEAN GIACOMOTTO ET AL: "High-throughput screening and small animal models, where are we?", BRITISH JOURNAL OF PHARMACOLOGY, WILEY-BLACKWELL, UK, vol. 160, no. 2, 4 March 2010 (2010-03-04), pages 204 - 216, XP071123591, ISSN: 0007-1188, DOI: 10.1111/J.1476-5381.2010.00725.X
- "IBMS-ECTS 2005 Abstracts ED - Karsenty Gerard; MacDougald Ormond; Rosen Clifford", BONE, PERGAMON PRESS., OXFORD, GB, vol. 36, 2005, pages S103 - S479, XP025396852, ISSN: 8756-3282, [retrieved on 20050101]
- FAN, W. G ET AL.: "ECM1 Prevents Activation of Transforming Growth Factor (3, Hepatic Stellate Cells, and Fibrogenesis in Mice", GASTROENTEROLOGY, vol. 157, no. 5, 27 July 2019 (2019-07-27), pages 1 - 29, XP085877366, DOI: 10.1053/j.gastro.2019.07.036
- OYAMA, N. ET AL: "The Extracellular Matrix Protein 1(ECM1) in Skin Biology: an Update for the Pleiotropic Action", THE OPEN DERMATOLOGY JOURNAL, vol. 7, 31 December 2013 (2013-12-31), pages 34 - 41, XP055705347
- LI, Z. H. ET AL.: "ECM1 Controls TH 2 Cell Egress from Lymph Nodes through Re-expression of S1P1", NATURE IMMUNOLOGY, vol. 12, no. 2, 1 February 2011 (2011-02-01), pages 180, XP055705359, ISSN: 1529-2908, DOI: 10.1038/ni.1983
- HE, L. ET AL: "Extracellular Matrix Protein 1 Promotes Follicular Helper T Cell Differentiation and Antibody Production", PNAS, vol. 115, no. 34, 21 August 2018 (2018-08-21), pages 8621 - 8626, XP055705362, ISSN: 0027-8424, DOI: 10.1073/pnas.1801196115

## Description

### Technical field

The present invention relates to the field of biomedicine, in particular to the use of ECM1 gene knockout mice in screening anti-hepatic fibrosis drugs.

### Background

Hepatic fibrosis is a dynamic process of liver damage repair, similar to wound repair reaction, which is characterized by abnormal accumulation of extracellular matrix in liver. Any factor that causes long-term chronic damage to the liver will induce this repair response. In China, the most common factor of liver injury is liver injury caused by chronic infection of HBV and HCV. Nonalcoholic fatty liver (NAFLD) is the most common pathogenic factor in developed countries. With the development of economic level and the emergence of excellent antiviral drugs and vaccines, the incidence of HBV and HCV is gradually decreasing, while the incidence and prevalence of nonalcoholic fatty liver are increasing. NAFLD is now recognized as the most common cause of chronic liver disease in the United States. It is believed that with the continuous development of China's economy, nonalcoholic fatty liver will also become the number one liver disease in China. Other causes of liver fibrosis include alcoholic liver disease, drug-induced liver disease, and liver disease caused by autoimmune, metabolism and biliary tract diseases.

The process of liver fibrosis is that the rate of collagen fiber formation in liver is greater than the rate of degradation. Although liver fibrosis can be reversed after eliminating injury, chronic persistent injury can lead to high crosslinking of extracellular matrix proteins and loss of potential reversibility. Liver cirrhosis is the final stage of fibrosis, with massive loss of liver parenchyma and severe distortion of vascular structure. Liver cirrhosis can lead to liver dysfunction, which is one of the important fatal diseases. The manifestations of decompensated liver cirrhosis include portal hypertension, variceal bleeding and hepatic encephalopathy which can lead to ascites. In addition, liver cirrhosis is a "precancerous condition". Over time, the risk of hepatocellular carcinoma (HCC) increases. Even in patients with advanced fibrosis who have not yet developed cirrhosis, the risk of HCC will increase.

At present, most animal models of liver fibrosis or its related diseases are induced animal models, which have many shortcomings, such as too long modeling time, no liver cirrhosis symptoms such as ascites or death, etc..

Therefore, there is an urgent need in the art to develop an animal model that can be used as a powerful tool for studying the mechanism of liver fibrosis and the screening of new drugs.

WO 2020/088070 A1 discloses an ECM1 knockout mouse as a model of liver fibrosis. Fan, Hepatology vol. 68, 2018:68;1-183 discloses that ECM1 KO mice die from spontaneous hepatic fibrogenesis.

### Summary of the Invention

An object of the present invention is to provide an animal model that can be used as a powerful tool for studying the mechanism of liver fibrosis and the screening of new drugs.

In a first aspect of the present invention, it provides a preparation method of an animal model of non-human mammal liver fibrosis or its related diseases, comprising the following steps:
(a) providing a cell of a non-human mammal, inactivating the *ECM1* gene in the cell, thereby obtaining a non-human mammalian cell with inactivated *ECM1* gene; and
(b) using the cell with inactivated *ECM1* gene obtained in step (a), preparing an animal model of liver fibrosis with an inactivated *ECM1* gene, wherein the animal model of liver fibrosis is an animal model of non-inducible spontaneous liver fibrosis, the non-human mammal is a mouse, the *ECM1* gene inactivation is systemic *ECM1* gene inactivation, and the animal model of non-human mammal liver fibrosis is homozygous and the animal model is a homozygous mouse with the age of 6-8 weeks;
   wherein compared with the wild-type control animal, the animal model of the non-human mammal with inactivated *ECM1* gene obtained in the step (b) has one or more of the characteristics selected from the group consisting of:
   (i) the increased progression of liver fibrosis;
   (ii) the increased number of α-SMA positive cells;
   (iii) the increased content of hydroxyproline;
   (iv) the increased expression of genes related to liver fibrosis;
   (v) the slightly increased content of aspartate aminotransferase (AST) and/or alanine aminotransferase (ALT);
   (vi) the increased activation of the stellate cell HSC; and/or
   (vii) the increased content of collagen.

**In** another preferred embodiment, the animal model of liver fibrosis or its related diseases is an animal model of early liver fibrosis.

**In** another preferred embodiment, the liver fibrosis or its related disease is an early liver fibrosis.

Step (a) may comprise the following steps:
(a1) using DNA homologous recombination technology, the coding region (such as exon 1 to exon 10) or non-coding region (such as 5'UTR or 3'UTR) or 3' coding region or intron region in the *ECM1* gene is deleted or gene-edited, and replaced with a selection marker to obtain a non-human mammalian cell with inactivated *ECM1* gene.

Disclosed herein but not forming part of the claimed invention, in step(b), further comprising the following steps:
(bl) using the non-human mammalian cell with inactivated *ECM1* gene obtained in step (a) to prepare a chimeric non-human mammal;
(b2) mating and breeding the chimeric non-human mammal obtained in step (b1) with a normal wild-type non-human mammal, and screening the offspring to obtain a heterozygous non-human mammal with inactivated *ECM1* gene; and
(b3) by mating the heterozygous non-human mammals obtained in step (b2) with each other, a homozygous non-human mammal with inactivated *ECM1* gene is obtained, thereby obtaining an animal model of a non-human mammal with inactivated *ECM1* gene.

Also disclosed herein but not forming part of the claimed invention, in step (b3), further comprising the step (b4): hybridizing the homozygous non-human mammal with inactivated *ECM1* gene with the liver-specific knock-out tool non-human mammal of the same species or the whole-body knock-out tool non-human mammal of the same species to obtain a non-human mammal animal model with liver-specific ECM1 gene inactivation or systemic ECM1 gene inactivation.

The inactivation of the *ECM1* gene may include gene knockout, gene interruption or gene insertion.

The gene inactivation may comprise no expression of *ECM1* gene, or the expression of an inactive *ECM1* protein.

The inactivation of the *ECM1* gene may also comprise reducing the expression of the *ECM1* gene or the protein thereof by 50%, preferably, 70%, more preferably, 80%, more preferably, 90%, more preferably, 100%.

The inactivation of the *ECM1* gene may further comprise the decrease in the expression level of the *ECM1* gene or the protein thereof is ≥5%, preferably ≥ 10%, more preferably, ≥ 20%, more preferably, ≥30% relative to a normal individual.

Also disclosed herein, the *ECM1* gene inactivation is liver-specific *ECM1* gene inactivation.

Also disclosed herein but not forming part of the claimed invention, in step (b4), mating a ECM1 Loxp/Loxp mouse with a tool mouse NSE (neuron-specific enolase)-Cre to obtain a liver-specific *ECM1* knockout mouse, referred to as a Alb-cre/ECM1^{flox/flox} mouse (i.e., a liver-specific ECM1 inactivated mouse).

Also disclosed herein but not forming part of the claimed invention, in step (b4), mating an ECM1 gene knockout heterozygous mouse with an ECM1 gene knockout heterozygous mouse to obtain a systemic ECM1 gene knockout mouse, referred to as an ECM1-KO mouse (i.e., a systemic ECM1 inactivated mouse).

The screening marker may be selected from the group consisting of a resistance gene, a fluorescent protein gene, and a combination thereof.

The screening marker may comprise *neo* gene and/or *GFP* gene.

The increase in the expression of genes related to liver fibrosis means that the expression of genes related to liver fibrosis may be ≥500, preferably ≥800, and more preferably, ≥1000 relative to a wild-type control animal.

The related gene of liver fibrosis may be selected from the group consisting of α-SMA, Col1a1, Col1a3, Desmin, and a combination thereof.

Also disclosed herein is the use of a mouse model prepared by the method according to the first aspect of the present invention, which is used as an animal model for studying liver fibrosis

Preferably, the animal model of liver fibrosis is an animal model of early liver fibrosis.

Preferably, the liver fibrosis is an early liver fibrosis

Also disclosed herein is the use of a mouse model prepared by the method according to the first aspect of the present invention for screening or identifying a substance (therapeutic agent) that can alleviate or treat liver fibrosis

Preferably, the liver fibrosis is an early liver fibrosis

Also disclosed herein is a method of screening or identifying a potential therapeutic agent for preventing and/or treating liver fibrosis, comprising the steps of:
(a) in a test group, in the culture system, in the presence of a test compound, culturing a cell expressing *ECM1* for a period of time T1, and detecting the expression level E1 of the *ECM1* gene or the protein thereof in the culture system of the test group; and/or the activity level V1 of the ECM1 protein;
   and in a control group in which the test compound is not present and other conditions being the same, detecting the expression level E2 of *ECM1* gene or the protein thereof in the culture system of the control group; and/or the activity level V2 of ECM1 protein; and
(b) comparing E1, E2, and/or V1, V2 detected in the previous step, thereby determining whether the test compound is a potential therapeutic agent for preventing and/or treating liver fibrosis; wherein if E1 is significantly higher than E2; and/or V1 is significantly higher than V2, indicating that the test compound is a potential therapeutic agent for preventing and/or treating liver fibrosis.

Preferably, the "significantly higher" refers to E1/E2≥2, preferably, ≥3, more preferably, ≥4.

Preferably, the "significantly higher" refers to V1/V2≥2, preferably, ≥3, more preferably, ≥4.

Preferably, the method is non-diagnostic and non-therapeutic.

Preferably, the method comprises the step (c), administering the potential therapeutic agent determined in step (b)to the mouse model prepared by the method of claim 1 to determine its effect on liver fibrosis in the animal model.

Preferably, the liver fibrosis is an early liver fibrosis

Also disclosed herein is a method of screening or identifying a potential therapeutic agent for preventing and/or treating liver fibrosis, comprising the steps of:
(a) in a test group, in the presence of the test compound, administering the test compound to a mouse model prepared by the method of claim 1, detecting the severity Q1 of liver fibrosis of the animal model in the test group; and in a control group to which the test compound is not administered and other conditions being the same, detecting the severity Q2 of liver fibrosis of the animal model in the control group; and
(b) comparing the severity Q1 and the severity Q2 detected in the previous step, thereby determining whether the test compound is a potential therapeutic agent for the prevention and/or treatment of liver fibrosis ;
wherein if the severity Q1 is significantly lower than the severity Q2, indicating that the test compound is a potential therapeutic agent for the prevention and/or treatment of liver fibrosis.

Preferably, the detection of the severity of liver fibrosis includes the detection of changes in one or more indicators selected from the group consisting of: the number of activated HSC, the number of α-SMA positive cells, the content of hydroxyproline, the content of alanine aminotransferase, the content of aspartate aminotransferase, collagen content in the liver, collagen fiber content in the liver.

Preferably, the reduction in the severity of liver fibrosis is manifested as: reduction in the progression degree of liver fibrosis in non-human mammals (such as mice), reduction in the number of α-SMA positive cells, reduction in the content of hydroxyproline, and reduction in the expression of genes related to liver fibrosis, slight reduction in the content of aspartate aminotransferase (AST) and/or alanine aminotransferase (ALT), reduction in the activation degree of stellate cell HSC, and reduction in the collagen content.

Preferably, the "significantly lower" means that the ratio of severity Q1/severity Q2 is ≤ 1/2, preferably ≤ 1/3, more preferably, ≤ 1/4.

Preferably, the method is non-diagnostic and non-therapeutic.

Preferably, the method comprises the step (c), administering the potential therapeutic agent screened or identified in step (b) to the mouse model prepared by the method of claim 1 to determine its effect on the severity of liver fibrosis in the animal model.

Preferably, the liver fibrosis or its related disease is an early liver fibrosis or its related disease.

Also disclosed herein is a mouse model **prepared** by the method according to the first aspect of the present invention.

For *ECM1* gene inactivation, the non-human mammalian model is homozygous.

The *ECM1* gene inactivation is systemic *ECM1* gene inactivation.

Also disclosed herein is the use of a cell in which the *ECM1* gene in the cell is inactivated or down-regulated for the preparation of a biological preparation for constructing an animal model of liver fibrosis in mice

Preferably, the biological preparation is a liquid preparation.

Preferably, the mouse model of liver fibrosis is an animal model of early liver fibrosis.

Preferably, the liver fibrosis is an early liver fibrosis

Also disclosed herein is the use of an inactivating agent or down-regulating agent of *ECM1* gene or a protein thereof for preparing a preparation for constructing an animal model of non-human mammal liver fibrosis or its related disease.

Preferably, the inactivating agent comprises an inhibitor.

Preferably, the inactivating agent or down-regulating agent refers to reducing the expression of the ECM1 gene or the protein thereof by 50%, preferably, 70%, more preferably, 80%, more preferably, 90%, more preferably, 100%.

Preferably, the inactivating agent or down-regulating agent refers to reducing the expression of the *ECM1* gene or the protein thereof by ≥5%, preferably, ≥ 10%, more preferably, ≥20%, more preferably, ≥30%.

Preferably, the inactivating agent or down-regulating agent is selected from the group consisting of an antibody, a small molecule compound, a nucleic acid, and a combination thereof.

Preferably, the mouse model of liver fibrosis is an animal model of early liver fibrosis**.**

Preferably, the liver fibrosis or its related disease is an early liver fibrosis or its related disease.

### Description of Drawings

Figure 1 shows phenotypic changes in ECM1 gene knockout mouse. ECM1 gene expression correct mice (WT) and ECM1 expression-deficient mice (KO) from the same parent in the same litter. Both mice are 8 weeks old.
Figure 2 shows changes in the liver of ECM1 gene knockout mice. The livers of ECM1 gene expression correct mice (WT) and ECM1 expression-deficient mice (KO) from the same parent in the same litter. Both mice are 8 weeks old.
Figure 3 shows staining of liver sections of ECM1-WT and KO mice. ECM1-WT and KO mice are sacrificed at the corresponding weeks of age (6 weeks and 8 weeks), and the livers are fixed, dehydrated, embedded and sectioned. H&E; Masson staining and a-SMA immunohistochemical staining are performed respectively.
Figure 4 shows detection of liver fibrosis markers in ECM1-WT and KO mice. Wherein
   (A) ECM1-WT and KO mice are sacrificed at the age of 8 weeks, the livers are taken for lysis, the samples are processed and detected according to the hydroxyproline detection kit, and finally the value is read at OD550 wavelength with a spectrophotometer.
   (B) Livers of 8-week-old ECM1-WT and KO mice are perfused, digested, sorted by density gradient centrifugation to obtain HSC. It is lysed with TRIZO, mRNA is extracted, and cDNA is obtained by reverse transcription. Finally the expression of related genes is detected by RT-PCR.

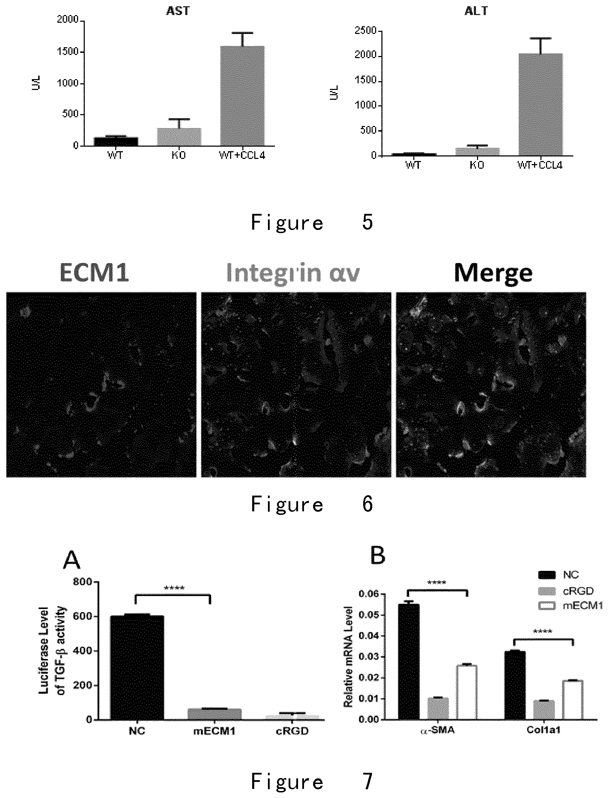

Figure 5 shows an analysis of liver injury in ECM1-KO mice. Wherein ECM1-WT and KO mice are sacrificed at 8 weeks old, and serum is collected. At the same time, a group of WT mice is taken and injected with CCL4 twice a week for modeling for 4 weeks, and then serum is taken as positive control. Serum samples are detected with corresponding AST and ALT detection kits.
Figure 6 shows the interaction of ECM1 protein with integrin αv. Wherein wild-type mouse (WT) liver is fixed with PFA, dehydrated, embedded, frozen sectioned, stained with anti-mouse ECM1 antibody and anti-integrin αv, then counterstained with DAPI, mounted, and photographed. Taking pictures with a laser confocal fluorescence microscope.
Figure 7 shows that ECM1 protein inhibits TGF-1 activation and mouse HSC activation. Wherein
   A: Primary stellate cells (HSC) from the liver of wild-type mice (WT) are isolated and co-cultured with NIH-3T3 cells containing the TGF-β1 activity reporter system. Recombinant mouse ECM1 protein (50g/ml), cRGD (10g/ml) or unrelated IgG protein (50g/ml) are added to the culture medium as negative control (NC). After 16 hours of the culture, the cells are lysed, and Luciferase activity in the lysate is detected.
   B: Primary stellate cells (HSC) from the liver of wild-type mouse (WT) are isolated and cultured *in vitro* for 2 weeks. Recombinant mouse ECM1 protein (50g/ml), cRGD (10g/ml) or unrelated IgG protein (50g/ml) are added to the culture medium as negative control (NC). Changing fresh culture medium every 3 days. Two weeks later, It is lysed with TRIZO and mRNA is extracted, and cDNA is obtained by reverse transcription. Finally, the expression of related genes is detected by RT-PCR.

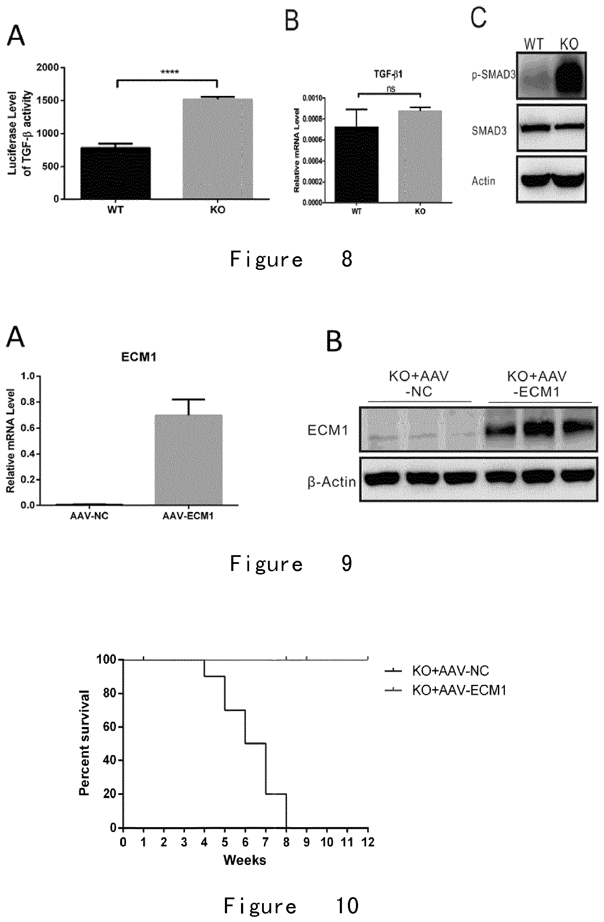

Figure 8 shows overactivation of TGF-β1 in the liver of ECM1 knockout mice (ECM1-KO). Wherein
   A: Primary stellate cells (HSC) from the liver of wild-type mice (WT) and ECM1 systemic knockout mice (KO) are isolated and co-cultured with NIH-3T3 cells containing TGF-β activity reporting system. After 16 hours of co-cultivation, the cells are lysed, and Luciferase activity in the lysate is detected.
   B: ECM1-WT and KO mice are sacrificed at the age of 8 weeks. The liver is taken for TRIZO lysis, the mRNA is extracted, and the cDNA is obtained by reverse transcription. Finally, the expression of TGF-mRNA is detected by RT-PCR.
   C: Primary stellate cells (HSC) from the liver of wild-type mice (WT) and ECM1 systemic knockout mice (KO) are isolated and lysed in RIPA lysate. After quantification with BCA, it is uniformly diluted to 2ug/ml with RIPA lysate, and then heated and denatured by adding Loading Buffer. When loading, each sample is 20ug/lane. The detection is performed using anti-phosphorylated SMAD3 (p-SMAD3) antibody, SMAD3 antibody and anti-Actin antibody.
FIG. 9 shows ECM1 gene re-expression in the liver of ECM1 systemic knockout mice (ECM1-KO) mediated by adeno-associated virus. Wherein
   A: ECM1 systemic knockout mice (ECM1-KO) are divided into two groups at the 4th week after birth and the 2/8 type adeno-associated virus (AAV-ECM1) expressing ECM1 gene or the control virus (AAV-NC) that does not express the gene is injected 1x10¹¹ through the tail vein. Four weeks after virus injection, the mice are sacrificed, the liver is collected, lysed with TRIZO, the mRNA is extracted and the cDNA is obtained by reverse transcription. Finally, the expression of ECM1 gene is detected by RT-PCR. The expression of GAPDH is used as internal reference.
   B: ECM1 systemic knockout mice (ECM1-KO) is injected with 1x10¹¹ of 2/8 type adeno-associated virus (AAV-ECM1) expressing ECM1 gene or control virus (AAV-NC) without gene expression via tail vein at the 4th week after birth. Four weeks after the virus injection, the mice are sacrificed, and 500mg of liver is collected and lysed in 1ml RIPA lysate, grinding and lysing. After quantification with BCA, it is uniformly diluted to 2ug/ml with RIPA lysate, and then heated and denatured by adding Loading Buffer. When loading, each sample is 20ug/lane. Anti-mouse ECM1 antibody is used for detection. The expression of Actin is used as an internal reference.
Figure 10 shows that adeno-associated virus mediated ECM1 expression prevents ECM1 systemic knockout mice (ECM1-KO) from death. Wherein ECM1 systemic knockout mice (ECM1-KO) are divided into two groups at the 4th week after birth, with 10 mice in each group. 1x10¹¹ 2/8 type adeno-associated virus (AAV-ECM1) expressing ECM1 gene or control virus (AAV-NC) without gene expression are injected via tail vein, respectively. The mortality of mice is observed and counted.

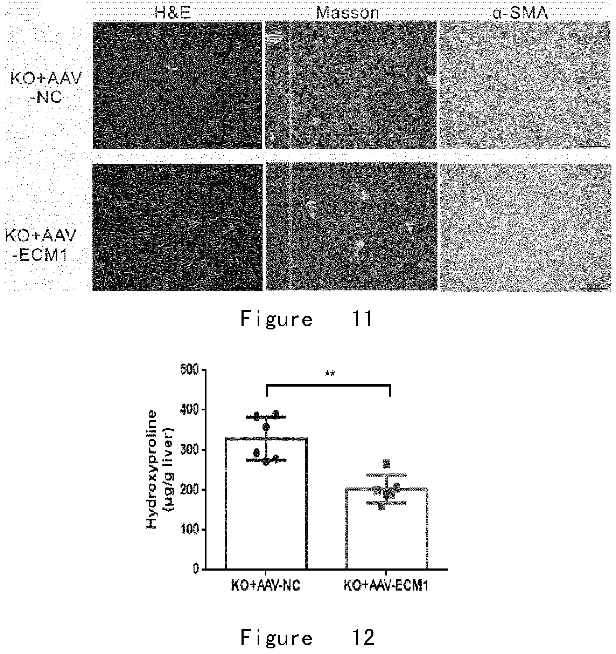

Figure 11 shows that adeno-associated virus mediated ECM1 expression inhibits liver fibrosis in ECM1 systemic knockout mice (ECM1-KO). Wherein ECM1 systemic knockout mice (ECM1-KO) are divided into two groups at the 4th week after birth, with 10 mice in each group. 1x10¹¹ 2/8 type adeno-associated virus (AAV-ECM1) expressing ECM1 gene or control virus (AAV-NC) without gene expression are injected via tail vein, respectively. Four weeks after virus injection, mice are sacrificed and their livers are fixed, dehydrated, embedded and sectioned. H&E; Masson staining and a-SMA immunohistochemical staining are performed respectively.
Figure 12 shows that adeno-associated virus mediated ECM1 expression inhibits liver fibrosis in ECM1 systemic knockout mice (ECM1-KO). Wherein ECM1 systemic knockout mice (ECM1-KO) are divided into two groups at the 4th week after birth, with 10 mice in each group. 1x10¹¹ 2/8 type adeno-associated virus (AAV-ECM1) expressing ECM1 gene or control virus (AAV-NC) without gene expression are injected via tail vein, respectively. Four weeks after the virus injection, the mice are sacrificed and the liver is lysed. The samples are processed and detected according to the hydroxyproline detection kit. Finally, the value is read at OD550 wavelength by spectrophotometer.

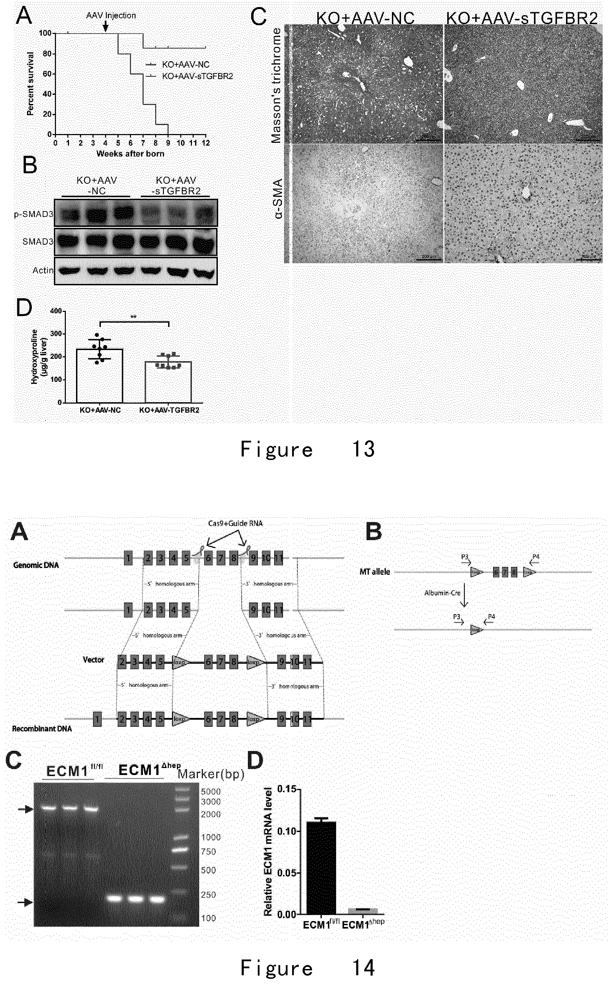

Figure 13 shows that adeno-associated virus-mediated expression of sTGFBR2 inhibits liver fibrosis in ECM1 systemic knockout mice (ECM1-KO). ECM1 systemic knockout mice (ECM1-KO) are divided into two groups at the 4th week after birth, with 10 mice in each group. The type 2/8 adeno-associated virus (AAV-sTGFBR2) of sTGFBR2 gene or the control virus (AAV-NC) that does not express the gene are injected through the tail vein, respectively. Wherein
   A: Observation and statistics of mice mortality.
   B: The mice are sacrificed, and 500mg of the liver is collected and lysed in 1ml of RIPA lysis solution, grinding and lysing. After quantification with BCA, it is uniformly diluted to 2ug/ml with RIPA lysate, and then heated and denatured by adding Loading Buffer. When loading, each sample is 20ug/lane. Anti-mouse smad3/p-smad3 antibody is used for detection. The expression of Actin is used as an internal reference.
   C: The mice are sacrificed and the liver is fixed, dehydrated, embedded and sectioned. Masson staining and a-SMA immunohistochemical staining are performed respectively.
   D: The mice are sacrificed and the liver is lysed. The samples are processed and detected according to the hydroxyproline detection kit. Finally, the value is read at OD550 wavelength by spectrophotometer.
Figure 14 shows the construction of hepatocyte-specific ECM1- conditional knockout mice.
   A and B: Showing the constructed ECM1 ^{F1/fl} and Albumin-Cre/ECM1^{fl/fl} (ECM1^{Δ hep}) mice.
   C: Identifying ECM1^{Δhep} mice with ECM1 gene knockout in hepatocytes and ECM1^{fl/fl} mice with normal expression of ECM1.
   D: Relative mRNA levels in the liver of ECM1 ^{Δhep} mice with ECM1 gene knockout in hepatocytes and ECM1^{fl/fl} mice with normal expression of ECM1.

### DETAILED DESCRIPTION

After extensive and in-depth research, the present inventors have unexpectedly discovered for the first time that decreasing the expression level of ECM1 gene in the liver or the whole body will lead to increased progression of liver fibrosis or its related diseases in mice. In addition, the present invention also establishes an animal model of liver fibrosis or its related diseases for the first time, which is a mouse whose ECM1 gene has been deleted or inactivated (including partial inactivation). The animal model of the present disclosure is an effective animal model of liver fibrosis, which can be used for studying liver fibrosis, and can be used for screening and testing of specific drugs. On this basis, the present inventors have completed the present invention.

### ECM1 gene and the protein thereof

As use herein, terms "ECM1", "Extracellular Matrix Protein 1" and "extracellular matrix protein 1" can be used interchangeably.

It should be understood that the term "ECM1" also includes various naturally occurring variants of the ECM1 gene. Representative examples include a nucleotide sequence encoding the same ECM1 protein as the wild type due to the degeneracy of the codon, and a nucleotide sequence encoding a conserved variant polypeptide of the wild type ECM1 protein. In addition, for mammals other than mice, the term refers to the homologue of ECM1 gene in the mammal. For example, for humans, the term refers to human ECM1 (it is known that the cDNA homology degree of mouse ECM1 gene and human ECM1 gene is 76.6%, and the homology degree of amino acid sequence is 73.4%). The accession number of mouse ECM1 gene: NM_007899.3; the accession number of mouse ECM1 protein : NP_031925.2, and the accession number of human ECM1 gene : NM_004425.3; the accession number of human ECM1 protein : NP_004416.2.

In the present invention, lack of ECM1 results in increased progression degree of liver fibrosis in mice, an increase in the number of α-SMA positive cells, an increase in the content of hydroxyproline, an increase in the expression of genes related to liver fibrosis, and a slight increase in the content of aspartate aminotransferase (AST ) and/or alanine aminotransferase (ALT), an increase in activation degree of stellate cells HSC, and an increase in the collagen content.

### Inactivating agent or down-regulating agent of ECM1 gene or a protein thereof

In the present disclosure, the inactivating agent of ECM1 protein includes complete inactivation or partial inactivation.

The inactivating agent of the ECM1 protein may include (a) an inhibitor, examples of the inhibitor include (but are not limited to): a small molecule compound, an antibody, an antisense nucleic acid, miRNA, siRNA, or a combination thereof; and/or (b) a knockout agent for ECM1 gene.

In the present disclosure, the inactivating agent or down-regulating agent refers to reducing the expression of the *ECM1* gene or the protein thereof by 50%, preferably, 70%, more preferably, 80%, more preferably, 90%, and more preferably, 100%.

### Gene inactivation

Many methods may be used to study genes with unknown functions, such as inactivating the gene to be studied, analyzing the phenotypic changes of genetic modifications, and then obtaining the functional information of the gene. Another advantage of this research method is that it may associate gene function with diseases. In this way, while gaining gene function, it can also obtain disease information and disease animal models that can be treated by the gene as a potential drug or drug target. The method of gene inactivation can be accomplished by gene knockout, gene interruption or gene insertion. Among them, gene knockout technology is a very powerful means to study the function of human genes in the whole.

In the present disclosure, gene inactivation further comprises reducing the expression of the *ECM1* gene or the protein thereof by 50%, preferably, 70%, more preferably, 80%, more preferably, 90%, and more preferably, 100%.

### Animal model

In the present invention, a very effective mouse model of liver fibrosis is provided.

As used herein, the term " ECM1 gene inactivation" includes the inactivation of one or two ECM1 genes, that is, including heterozygous and homozygous inactivation of ECM1 gene. For example, ECM1 gene inactivated mice may be heterozygous or homozygous.

In the present disclosure, ECM1 gene inactivated mice may be prepared by methods such as gene knockout or transfer of exogenous genes (or fragments) to inactivate the ECM1 gene. In the present art, techniques for inactivating target genes by gene knockout or transfer of exogenous genes are known, and all these conventional techniques may be used in the present invention.

The inactivation of ECM1 gene may be achieved through gene knockout.

The inactivation of ECM1 gene may be achieved by inserting an exogenous gene (or fragments) into the ECM1 gene.

In a specific embodiment of the present disclosure, a construct containing exogenous insertion fragments may be constructed. The construct contains homology arms that are homologous to the flanking sequences on both sides of the insertion site of the target gene (ECM1). Therefore, the exogenous insertion fragments (or gene) may be inserted into the ECM1 genome sequence (especially the exon region) at a high frequency through homologous recombination, which causes the frameshift, premature termination, or knockout of the mouse ECM1 gene, resulting in the deletion or inactivation of the ECM1 gene.

Homozygous or heterozygous mice obtained by the method disclosed herein are fertile. The inactivated ECM1 gene can be inherited to offspring mice by Mendelian law.

The present disclosure also provides a homozygous mouse model animal lacking ECM1 gene.

The disclosure also provides a hepatocyte-specific ECM1 conditional knockout mouse model animal is provided.

In the present invention, the mouse model of liver fibrosis of the present invention may be an animal model of early liver fibrosis. For example, in mice, liver fibrosis appears within 5-6 weeks. In humans, liver fibrosis begins 1 week after the onset of liver fibrosis.

### Candidate drug or therapeutic agent

In the present disclosure, there is also provided a method for screening a candidate drug or therapeutic agent for the treatment of liver fibrosis by using the mouse model of the present invention.

In the present disclosure, a candidate drug or therapeutic agent refers to a substance that is known to have certain pharmacological activities or is being detected and may have certain pharmacological activities, including but not limited to a nucleic acid, a protein, saccharides, a chemically synthesized small or macromolecular compound, a cell, and the like. The administration of the candidate drug or therapeutic agent can be oral, intravenous, intraperitoneal, subcutaneous, spinal or direct intracerebral injection.

### Drug screening method

The disclosure also provides a method for drug screening based on ECM1. One method is to first screen compounds that affect (enhance) ECM1 expression or activity, and then further test the screened compounds for their therapeutic effects on animal model mice suffering from liver fibrosis.

The method of screening therapeutic agents for preventing and/or treating liver fibrosis provided by the present disclosure, based on the effect of the compound on the expression level and/or activity of ECM1, a typical screening method comprises the steps of:
(a) in the test group, in the culture system, in the presence of a test compound, culturing a cell expressing *ECM1* for a period of time T1, and detecting the expression level E1 of the *ECM1* gene or the protein thereof in the culture system of the test group; and/or the activity level V1 of the ECM1 protein;
   and in the control group in which the test compound is not present and other conditions are the same, detecting the expression level E2 of *ECM1* gene or the protein thereof in the culture system of the control group; and/or the activity level V2 of ECM1 protein; and
(b) comparing E1, E2, and/or V1, V2 detected in the previous step, thereby determining whether the test compound is a potential therapeutic agent for preventing and/or treating liver fibrosis; wherein if E1 is significantly higher than E2; and/or V1 is significantly higher than V2, indicating that the test compound is a potential therapeutic agent for preventing and/or treating liver fibrosis.

Preferably, the method comprises the step (c), the potential therapeutic agent determined in step (b) is administered to the mouse model prepared by the method of the present invention to determine its therapeutic effect on liver fibrosis in the animal model.

The expression level of ECM1 can be carried out at mRNA level or protein level, for example, by conventional methods or commercially available equipment and reagents (such as antibodies, primers, etc.).

### The main advantages of the present invention include:

(1) The present invention has discovered for the first time that ECM1 plays an important role in maintaining liver homeostasis, and has discovered for the first time that the lack of ECM1 will lead to increased progression degree of liver fibrosis in mice, an increase in the number of α-SMA positive cells, an increase in the content of hydroxyproline, an increase in the expression of genes related to liver fibrosis, a slight increase in the content of an aspartate aminotransferase (AST) and/or alanine aminotransferase (ALT), an increase in activation of stellate cells HSC, and an increase in collagen content.
(2) The present invention has constructed a mouse model of liver fibrosis in non-human mammals for the first time, and the model can be used to effectively screen therapeutic agents for preventing and/or treating liver fibrosis .
(3) Through detailed research on ECM1 systematic knockout mice, the present invention has discovered for the first time that the mode of fibrosis occurrence is inconsistent with the classical liver fibrosis model, and is a brand-new and unique spontaneous liver fibrosis model.
(4) The present invention has discovered for the first time that extracellular matrix protein 1 (ECM1) is expressed in a large amount in the extracellular matrix of the liver, and systemic knockout of ECM1 will lead to spontaneous, severe and fatal liver fibrosis in mice.
(5) The present invention has discovered for the first time that ECM1 protein inhibits HSC activation and collagen production by interacting with integrin αv molecule.
(6) The present invention has discovered for the first time that re-expression of ECM1 gene in the liver of ECM1 gene knockout mice can also inhibit the occurrence of liver fibrosis.
(7) The present invention has discovered for the first time that ECM1 gene knockout mice can spontaneously develop liver fibrosis diseases, and is a very unique liver fibrosis mouse model, which can be used for screening and verification of anti-liver fibrosis drugs.

The present invention will be further explained below in conjunction with specific embodiments. It should be understood that these embodiments are only used to illustrate the present invention and not to limit the scope of the present invention. The experimental methods without specific conditions in the following examples are usually in accordance with conventional conditions such as Sambrook et al., Molecular Cloning: Laboratory Manual (New York: Cold Spring Harbor Laboratory Press, 1989), or in accordance with the conditions described by the manufacturer The suggested conditions. Unless otherwise stated, percentages and parts are calculated by weight.

Unless otherwise specified, all materials and reagents used in the examples are commercially available.

### Experimental method

### Construction of ECM1 gene knockout mice

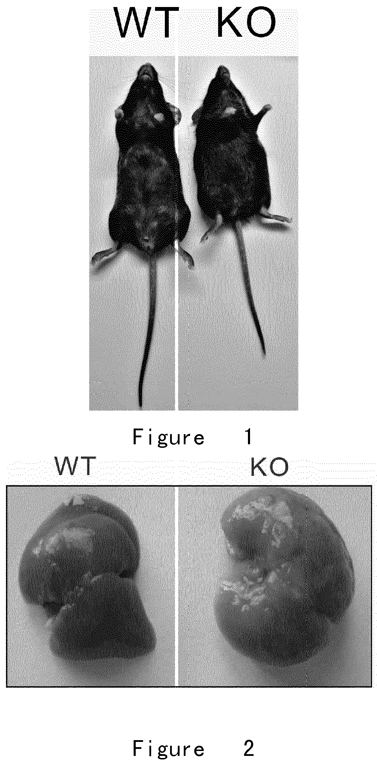

Basic steps of constructing gene knockout animal model by homologous recombination (Figure 1):
①. Construction of gene vector: Recombinate the target gene and the DNA molecule homologous to the specific fragment of the target gene in the cell into the vector with the marker gene (such as neo gene, TK gene, etc.) to form a recombination vector. Gene knockout is to make a gene lose its physiological function, so it is generally designed as a replacement vector. [1]
②. Acquisition of ES cells: At present, embryonic stem cells are generally used for gene knockout, most commonly mice, and embryonic stem cells from rabbits, pigs, chickens, etc. are also used. The commonly used mouse germline is 129 and its heterozygotes, because these mice have the tendency of spontaneous mutation to form teratoma and teratosarcoma, and are ideal experimental animals for gene knockout. Embryonic stem cell lines with other genetic backgrounds have gradually been developed and applied. [2, 3]
③. Homologous recombination: The recombinant vectors are introduced into homologous embryonic stem cells (ES cells) by a certain method (electroporation or microinjection), so that the exogenous DNA and the corresponding part of the embryonic stem cell genome will undergo homologous recombination, the DNA sequence in the recombinant vector is integrated into the endogenous genome, thus to be expressed. Generally, the hit rate of microinjection is higher, but the technique is more difficult. The hit rate of electroporation is lower than that of microinjection, but it is easy to use. [4,5]
④. Selecting and screening the hit cells: Because the natural incidence of homologous recombination of gene transfer is extremely low, the recombination probability of animals is 10-2 ~10-5, the probability of plants is 10-4 ~10-5. Therefore, it is very important to screen out embryonic stem cells that have undergone homologous recombination from many cells. At present, the commonly used method is positive and negative screening method (PNS Method), marker gene specific site expression method and PCR method. Among them, the most widely used is PNS method. [6]
⑤. Phenotypic research: By observing the changes of biological shape of chimera mice, we can understand the changes of biological shape of mice before and after the changes of target genes, so as to achieve the purpose of studying target genes. [2, 3,7]
⑥. Homozygote acquisition: Since homologous recombination often occur in one of a pair of chromosomes. Therefore, if a stable genetic homozygous gene knockout model is to be obtained, at least two generations of inheritance are required.

### Construction of ECM1 gene liver conditional knockout mice

Homozygous ECM1 hepatic parenchymatous cell-specific knockout mice (Alb cre/ECM1 flox/flox) were obtained by mating Alb cre mice with ECM1 flox/flox mice, as shown in A-D of Figure 14. By sorting different cells in the liver, mRNA was extracted, and reverse transcription was performed to obtain cDNA, and finally, the expression of ECM1 was detected by RT PCR to confirm that the conditional knockout of ECM1 mediated by Alb cre only occured in hepatic parenchymal cells, while ECM1 gene expression in the liver of ECM1 flox/flox mice was normal. Therefore, ECM1 hepatic parenchymal cell-specific knockout mice (Alb cre/ ECM1 flox/ flox) can be used in liver fibrosis modeling experiments to compare the effects of ECM1 protein produced by hepatocytes on liver fibrosis.

### EXAMPLE1 Decreased expression of ECM1 gene promotes the development of liver fibrosis

ECM1 systematic knockout mice obtained from the present laboratory were found to die spontaneously when homozygous mice (ECM1-KO) developed to the age of 6-8 weeks during the breeding and culture process. The main manifestations were smaller body size than normal control mice (ECM1-WT), less subcutaneous fat, and suspected ascites due to abdominal bulging (Figure 1). Therefore, we conducted detailed pathological diagnosis and research on ECM1-KO mice to find the cause of spontaneous death of ECM1-KO mice.

### EXAMPLE2 Spontaneous liver fibrosis occurs in ECM1 systemic knockout mice (ECM1-KO)

After the mouse was dissected, it was found that most of the mouse organs were developed normally, and there was no obvious pathological change in naked eye observation. However, the abdominal cavity of mouse was filled with ascites, the liver became lighter in color, hard in texture and uneven in surface (Figure 2). The changes of liver and the appearance of ascites suggested that our mice may have severe liver fibrosis, and even progress to liver cirrhosis in the later stage.

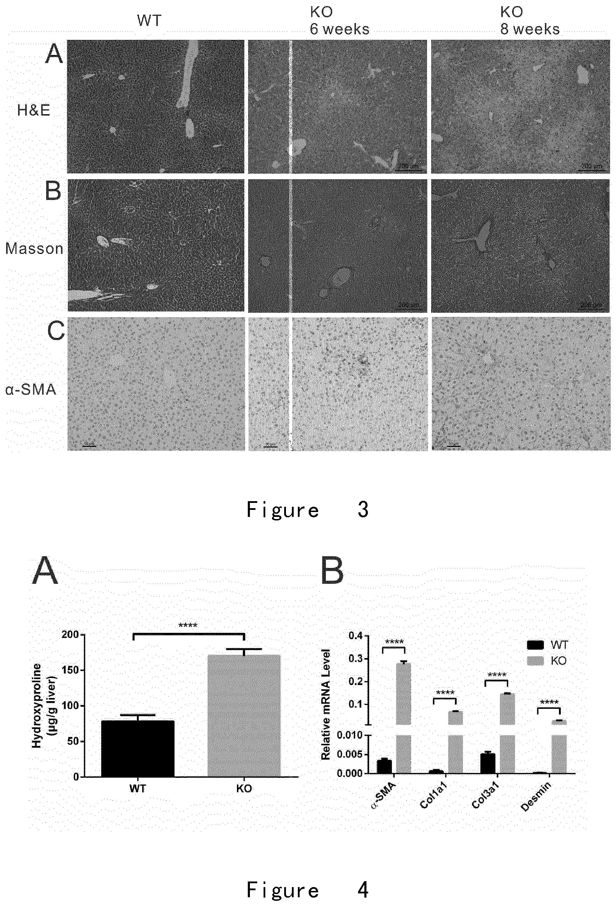

In order to further confirm whether liver fibrosis occurs in mice, the liver of mice was analyzed by section staining. We collected the liver of mice of different weeks of age for paraffin section staining. The results of H&E staining show that compared with WT mice of the same age, the liver structure of ECM1-KO mice has changed, the hepatic parenchymatous cell has decreased, and a large number of reddish substance deposits are appeared in the tissues (Figure 3A). Because a large amount of collagen will be deposited in the liver during liver fibrosis, in order to further determine whether the substance deposited in the liver is collagen, we performed Masson staining on the liver. Masson staining can specifically dye collagen blue. The staining results show that a large amount of collagen stained blue are deposited in the liver of ECM1-KO mice. Moreover, these collagen proteins are mainly deposited in the hepatic sinusoids. This deposition method is significantly different from the typical mouse liver fibrosis model created by CCL4 (Figure 3B). Moreover, this collagen deposition pattern is more likely to induce portal hypertension and ascites formation.

Another obvious sign of liver fibrosis is that a large number of quiescent HSC cells (qHSC) in the liver are activated into activated HSC (aHSC), which can produce a large amount of collagen, also known as fibroblasts (MFs). α-SMA is one of the typical markers of fibroblasts. The number of aHSC in liver can further reflect the progress of liver fibrosis.

After immunohistochemical staining of α-SMA in liver sections of ECM1-WT mice and KO mice, it was found that in WT mice, only the walls of large blood vessels were positive for α-SMA. However, in the liver sections of KO mice, a large number of α-SMA positive cells appears in the hepatic sinusoidal space (Figure 3C). And with the increase of the age of the mice, the α-SMApositive cellsare also increased, which is consistent with the changes in the results of Masson staining.

**In** order to further confirm the occurrence of liver fibrosis in ECM1-KO mice, we measured the amount of hydroxyproline (HYP) in the liver. Hydroxyproline is one of imino acids, a non-essential amino acid, and one of the main components of collagen tissue, and it is a unique amino acid in collagen, accounting for about 13% of the total amino acids in collagen. Taking advantage of the feature that hydroxyproline has the highest content in collagen, the metabolism of collagen in the liver can be understood by measuring hydroxyproline in the liver. The results of the hydroxyproline detection kit show that the content of hydroxyproline in the liver of ECM1-KO mice is much higher than that of WT mice, indicating that a large amount of collagen is deposited in the liver of ECM1-KO mice (Figure 4A).

Detecting the expression of some genes related to fibrosis in the liver is also a recognized method for the diagnosis of liver fibrosis. The genes commonly used for detection are stellate cell activation related genes (α-SMA, Desmin, etc.) already related collagen expression genes (Col1a1; Col1a3, etc.). We perfused the livers of 8-week-old ECM1-WT and KO mice, digested them, and separated them by density gradient centrifugation to obtain HSC. Lysed by TRIZO, mRNA was extracted, reverse transcription was performed to obtain cDNA, and finally the expression of related genes was detected by RT-PCR. The results show that the expression level of liver fiber-related genes in KO mice is more than 1000 times higher than that in ECM1-WT mice (Figure 4B).

These results indicate that the liver of ECM1-KO mice does have spontaneous fibrosis without any external stimulation.

### EXAMPLE3 Analysis of Liver Function of ECM1 Systemic Knockout Mice (ECM1-KO)

After preliminarily confirming that ECM1-KO mice did have severe spontaneous fibrosis, we conducted a preliminary literature search and comparedwith the existing gene knockout liver fibrosis model. The results show that most gene knockout mice still need external stimulation or induction to develop liver fibrosis, such as drug stimulation (CCL4, TAA, etc.), diet regulation (high fat diet, MCD diet, etc.) and bile duct ligation. Only a few gene knockout mice can develop spontaneous liver fibrosis without external stimulation, but these mice progress slowly in fibrosis and have similar progression patterns of liver fibrosis compared with induced mouse models, such as a large number of hepatocyte death and inflammatory cell activation.

In order to further compare the similarities and differences between the spontaneous liver fibrosis model of ECM1-KO mice and the classical liver fibrosis-induced model, the serum of ECM1-KO mice and CCL4-induced liver fibrosis model mice were collected, and the liver function (aspartate aminotransferase AST and alanine aminotransferase ALT) was tested. These two indexes can reflect the death of hepatocytes during the development of liver fibrosis.

The test results showe that when ECM1-KO mice are 8 weeks old with severe liver fibrosis and ascites, the values of AST and ALT are only slightly higher than those of WT mice, and much lower than CCL4-induced liver fibrosis model mice (Figure 5 ). These results suggest that the cause and mechanism of liver fibrosis in ECM1-KO mice are inconsistent with the classical liver fibrosis model.

### EXAMPLE4 ECM1 protein inhibits the activation of TGFβ1 and the activation of HSC by interacting with integrin αv

The study of ECM1 systemic knockout mice (ECM1-KO) and hepatic parenchymatous cell (Hepatocyte) conditional knockout mice (Alb-cre/ECM1 ^{Flox/flox}) shows that ECM1 gene knockout has no significant effect on hepatocyte injury or inflammation like other classical fibrosis models. ECM1 systemic knockout mice (ECM1-KO) and hepatic parenchymatous cell (Hepatocyte) conditional knockout mice (Alb-cre/ECM1 ^{Flox/flox}) both show that stellate cells (HSC) in the hepatic sinusoid are activated in situ and transformed into activated fibroblasts. In the process of stellate cell (HSC) activation, the most important and necessary factor is the stimulation of TGF-β1. Stellate cells (HSC) must be stimulated by TGF-1 to be activated and transformed into activated fibroblasts. If the function of TGF-β1 is inhibited in this process, the activation of stellate cells (HSC) will also be inhibited.

TGF-B1 can be synthesized by a variety of cells in the liver. However, the synthetic TGF-β1 is initially secreted outside the cell in an inactive form, which is called Latent TGF-β1. Latent TGF-β1 and LAP (latency-associated peptide) combine through LTBP1 (latent TGF-1 binding protein 1) to form LLC (large latent complex), which is stored in extracellular matrix. Latent TGF-β1 needs to be cleaved by specific factors outside the cell and isolated from LAP before becoming active TGF-β1, binding to TGF-β1 receptor and activating downstream signaling pathways. Integrins are mainly constitutively expressed on many kinds of cell surfaces. They are a family of heterodimeric receptor molecules composed of α subunits and β subunits. Up to now, 24 subunits have been found in integrin family, including 18 α subunits and 6 β subunits. In the integrin family of molecules, there are five integrin molecules containing αv subunits (αvβ1, αvβ3, αvβ5, αvβ6, and αvβ8). These five integrin molecules containing αv subunits can bind to the RGD (arginine-glycine-asparticacid) tripeptide sequence on the LAP in the TGF-β1 precursor complex. This binding is very important for the maturation and activation of the precursor TGF-β1 under physiological conditions. Subsequent studies found that αv integrin is necessary in the activation process of TGF-β1. The deletion of αv integrin or the mutation of αv integrin binding site on TGF-β1 will make the TGF-β1 precursor *in vivo* unable to mature and activate to produce bioactive TGF-β1, resulting in the deletion of TGF-β1 signaling pathway *in vivo.*

### 4.1 ECM1 protein interacts with Integrin αV

The interaction between ECM1 protein and integrin αV was determined by in situ fluorescence staining of mouse liver sections. The results of immunofluorescence staining show that ECM1 protein and integrin αV in mouse liver are strongly positive in hepatic sinusoids, and red fluorescence can be superimposed with green fluorescence to form yellow fluorescence signal, indicating that ECM1 protein interacts with integrin αV in the liver of mice (Figure 6).

### 4.2 ECM1 protein inhibits the activation of TGF-β1 and the activation of HSC in mouse stellate cells

The process of Latent TGF-β1 activation to TGF-β1 is a transient effect. Activated TGF-β1 will bind to TGF-β1 receptor on the adjacent cell surface, phosphorylating the downstream SMAD protein, and then activating the downstream gene expression. In order to better detect the process of Latent TGF-β1 activation to TGF-β1, we used a co-culture system to detect the TGFβ1β1 activation process that occurs on the surface of HSC stellate cells. A luciferase reporter gene was stably transfected into NIH-3T3 cells. There are 4 repeated SMAD binding sites in the upstream of this reporter gene. Therefore, once Latent TGF-β1 is activated to active TGF-β1, it will bind to the receptor on the surface of NIH-3T3 cells, activating the SMAD signaling pathway downstream of NIH-3T3 cells, and inducing luciferase reporter gene expression. By detecting the activity of luciferase in NIH-3T3 cells, it can reflect how much Latent TGF-β1 is activated to active TGF-β1 in this co-culture system.

Firstly, primary stellate cells (HSC) of wild-type mouse (WT) liver were isolated and co-cultured with NIH-3T3 cells containing TGF-β1 activity reporter system. Recombinant mouse ECM1 protein (50g/ml), cRGD (10g/ml) or unrelated IgG protein (50g/ml) were added to the culture medium as negative control (NC). After 16 hours of culture, the cells were lysed, and Luciferase activity in the lysate was detected. The experimental results show that the recombinant mouse ECM1 protein can significantly inhibit the activation of Latent TGF-β1 on the surface of stellate cells (HSC) (Figure 7A). In this system, we added a classic integrin αv inhibitor cRGD as a positive control.

The primary stellate cells (HSC) of the mouse liver will self-activate during *in vitro* culture. This is because of the change in culture conditions, the Latent TGF-β1 secreted by the cells will be activated by integrin αv on the cell surface to activate stellate cells (HSC). In this process, the addition of integrin αv inhibitor (cRGD) or TGF-β1 neutralizing antibody will inhibit the self-activation of stellate cells (HSC).

Therefore, primary stellate cells (HSC) from the liver of wild-type mice (WT) were isolated and cultured *in vitro* for 2 weeks. Recombinant mouse ECM1 protein (50g/ml), cRGD (10g/ml) or unrelated IgG protein (50g/ml) were added to the culture medium as negative control (NC). Changing fresh culture solution every 3 days. Two weeks later, TRIZO was used to lyse and mRNA was extracted, and cDNA was obtained by reverse transcription. Finally, the expression of related genes was detected by RT-PCR to determine the activation degree of primary stellate cells (HSC). The experimental results show that both the recombinant mouse ECM1 protein and cRGD cam significantly inhibit the activation of stellate cells (HSC) (Figure 7B).

These experimental results show that ECM1 protein has similar function to cRGD, that is to inhibit the Latent TGF-β1 in the extracellular matrix by the interaction with integrin αv, which will be activated to TGF-β1 by the integrin αv on the cell surface, thereby activating the stellate cells (HSC) in the hepatic sinusoid to transform to activated fibroblasts cell.

### 4.3 TGF-β1 is overactivated in the liver of ECM1 knockout mice (ECM1-KO).

In order to further confirm the pathogenesis of liver fibrosis in ECM1 systemic knockout mice (ECM1-KO), we detected TGF-β-signaling pathway in their liver in detail.

Firstly, primary stellate cells (HSC) from the livers of wild-type mice (WT) and ECM1 systemic knockout mice (KO) were isolated, and co-cultured with NIH-3T3 cells containing the TGF-β1 activity reporter system. After co-culturing for 16 hours, the cells were lysed, and Luciferase activity in the lysate was detected. The experimental results show that stellate cells (HSC) derived from the liver of ECM1 systemic knockout mice (KO) can produce more TGF-β1, indicating that the stellate cell (HSC) in the liver of ECM1 systemic knockout mouse (KO) has stronger collagen production ability and is already a very activated stellate cell (HSC) (Figure 8A).

In order to determine whether the strong TGF-β1 signal in the liver of ECM1 systemic knockout mice (KO) is due to the overexpression of TGF-β1 gene or the overactivation of Latent TGF-β1, we sacrificed ECM1-WT and KO mice at the age of 8 weeks. The liver was lysed by TRIZO, the mRNA was extracted, and the cDNA was obtained by reverse transcription. Finally, the expression of TGF-β1 mRNA was detected by RT-PCR. The experimental results show that the expression of TGF-β1 gene in the liver of ECM1 systemic knockout mice (KO) is not significantly different from that of ECM1-WT mice (Figure 8B).

At the same time, we also isolated primary stellate cells (HSC) from the liver of wild-type mice (WT) and ECM1 systemic knockout mice (KO), and lysed them in RIPA lysis buffer. After quantification with BCA, it was uniformly diluted to 2ug/ml with RIPA lysate, and then heated and denatured by adding Loading Buffer. When loading, each sample was 20ug/lane. The detection was performed using anti-phosphorylated SMAD3 (p-SMAD3) antibody, SMAD3 antibody and anti-Actin antibody. The experimental results show that the phosphorylation of SMAD3 in the liver of ECM1 systemic knockout mice (KO) is significantly enhanced (Figure 8C).

These experimental results indicate that ECM1 protein can be activated to TGF-β1 by inhibiting Latent TGF-β1 in extracellular matrix. At the same time, it can also inhibit the activation of stellate cells (HSC) and the production of collagen through this signaling pathway.

### EXAMPLES Adeno-associated virus-mediated ECM1 expression inhibits liver fibrosis in ECM1-KO mice

As a gene constitutively expressed in hepatic parenchymal cells, ECM1 gene is down-regulated during the process of liver fibrosis, thereby promoting the development of liver fibrosis. The inventors further studied whether re-expression of ECM1 gene in hepatic parenchymal cells can inhibit the development of liver fibrosis.

Adeno-associated virus (AAV) belongs to the family Parvoviridae, without coating and with an icosahedral structure. The genetic gene is a linear single-stranded DNA molecule with a size of about 4.7 kb, and its two ends are inverted repeat ITRs, each ITR is 145bp in size. The two open reading frames (ORF) in the middle of the two ITRs are Rep and Cap, respectively. Among them, Rep encodes 4 proteins and Cap encodes 3 protein capsids. Rep gene and Cap gene in recombinant adeno-associated virus were knocked out, and the target gene was loaded between ITR at the same time to form virus plasmid vector. Subsequently, the plasmid vector carrying the adenovirus helper gene and the AVV plasmid vector carrying the Rep gene with replication function and the Cap gene with transfection function were co-transfected into the same cell line to form a recombinant adeno-associated virus with transfection ability. AVV is divided into 13 serotypes AVV1 ~ AVV13. All AVV have an icosahedral structure. However, due to differences in molecular sequence and spatial configuration of different serotypes, the transfection affinity of different serotypes of AVV is also different. Studies have shown that AVV8 has a strong affinity for hepatocytes. Therefore, the AVV8 recombinant adeno-associated virus vector that specifically infects hepatic parenchymatous cells can be selected to overexpress the ECM1 gene in the liver.

### EXAMPLE6 Adeno-associated virus-mediated re-expression of ECM1 gene in the liver of ECM1-KO mice

Because the gene expression mediated by AVV8 recombinant adeno-associated virus takes 1-2 weeks after infection to reach the peak of expression, and ECM1 systemic knockout mice (ECM1-KO) do not begin to show obvious liver fibrosis symptoms until the 4th week after birth. Therefore, the ECM1 systemic knockout mice (ECM1-KO) obtained by our mating were divided into two groups at the 4th week after birth and 1 × 10¹¹ type 8 adeno-associated virus expressing ECM1 gene (AAV-ECM1) or a control virus without gene expression (AAV-NC) was injected through the tail vein, respectively. Four weeks after virus injection, the mice were sacrificed, the liver was collected, and the expression of ECM1 gene in the liver of mice was detected.

The experimental results show that after 4 weeks of injection of the recombinant adeno-associated virus AVV8, a very strong ECM1 gene mRNA transcription and protein expression can be detected in the liver of the AAV-ECM1 group, while ECM1 gene expression can not be detected in the negative control group (Figure 9, A and B).

### EXAMPLE7 Adeno-associated virus-mediated ECM1 expression protects ECM1-KO mice from death

Because ECM1 systemic knockout mice (ECM1-KO) will die of ascites and complications caused by severe liver fibrosis within 6-8 days after birth, we want to observe whether the re-expression of ECM1 gene mediated by AVV8 recombinant adeno-associated virus can save mice.

We divided ECM1 systemic knockout mice (ECM1-KO) into two groups at the 4th week after birth, with 10 mice in each group. 1x10¹¹ type 2/8 adeno-associated virus expressing ECM1 gene (AAV-ECM1) or a control virus without gene expression (AAV-NC) was injected through the tail vein, respectively. The mortality of mice was observed and counted. The experimental results show that the ECM1 systemic knockout mice (ECM1-KO) injected with the control virus without gene expression (AAV-NC) will die of ascites and complications caused by severe liver fibrosis within 6-8 after birth, as previously observed mice. However, all mice injected with type 2/8 adeno-associated virus expressing ECM1 gene (AAV-ECM1) survive (Figure 10).

### EXAMPLES Adeno-associated virus-mediated ECM1 expression inhibits liver fibrosis in ECM1-KO mice

At the same time, ECM1 systemic knockout mice (ECM1-KO) were divided into two groups at the 4th week after birth, and 1x10¹¹ type 2/8 adeno-associated virus expressing ECM1 gene (AAV-ECM1) or a control virus without gene expression (AAV-NC) was injected through the tail vein, respectively. Four weeks after virus injection, mice were sacrificed and their livers were fixed, dehydrated, embedded and sectioned. H&E; Masson staining and α-SMA immunohistochemical staining were performed respectively.

The experimental results of H&E staining show that the liver structure of the ECM1 systemic knockout mice (ECM1-KO) injected with the control virus without gene expression (AAV-NC)has changed, hepatic parenchymatous cells were reduced, and there is a large amount of material deposition dyed light red in the tissue, while the liver structure of mice injected with 2/8 adeno-associated virus expressing ECM1 gene (AAV-ECM1) is similar to normal mice. Masson staining results show that the content of blue collagen in the liver of mice injected with type 2/8 adeno-associated virus (AAV-ECM1) expressing ECM1 gene is greatly reduced. Similarly, in the liver sections of mice injected with type 2/8 adeno-associated virus expressing ECM1 gene (AAV-ECM1), a large number of α-SMA positive cells appears in the hepatic sinusoidal space. However, the liver structure of mice injected with type 2/8 adeno-associated virus expressing ECM1 gene (AAV-ECM1) is similar to that of normal mice, only the walls of large blood vessels are positive for a-SMA (Figure 11). The results of the hydroxyproline detection kit show that the hydroxyproline content in the liver of mice injected with the type 2/8 adeno-associated virus expressing the ECM1 gene (AAV-ECM1) is much lower than that in the (AAV-NC) group mice (Figure 12).

These experimental results indicate that using the type 2/8 adeno-associated virus to mediate the re-expression of the ECM1 gene in the liver can protect the liver of ECM1 systemic knockout mice (ECM1-KO) from spontaneous fibrosis. These experimental results indicate that the ECM1 protein can inhibit the activation of stellate cells and the production of liver fibrosis in mice. At the same time, it also shows that in ECM1 systemic knockout mice (ECM1-KO), severe liver fibrosis and its complications are the most important cause of death in ECM1-KO mice.

### EXAMPLE9 Blocking the TGF-β1 signaling pathway in the liver of ECM1-KO mice can inhibit liver fibrosis in ECM1-KO mice

sTGFBR2 is an extracellular soluble fragment of TGF-β1 receptor, which has been proved to be used to bind to TGF-β1 *in vivo* and inhibit the activity and function of TGF-β1. In the present invention, re-expressing the sTGFBR2 gene in hepatic parenchymatous cell can inhibit the development of liver fibrosis. Once again, it is proved that blocking the TGF-β1 signaling pathway in the liver of ECM1-KO mice can inhibit liver fibrosis in ECM1-KO mice (Figure 13).

### Discussion

The above experimental results indicate that ECM1 protein can inhibit the activation of Latent TGF-β1 in the extracellular matrix to TGF-β1. Therefore, ECM1 protein in liver extracellular matrix can inhibit the activation of stellate cells (HSC) and the production of collagen.

Therefore, in the liver of ECM1 systemic knockout mice (KO), a large number of Latent TGF-β1 is activated to TGF-β1, and silenced stellate cells are activated to fibroblasts expressing a large amount of collagen, resulting in spontaneous liver fibrosis in the liver of ECM1 systemic knockout mice (KO). However, in ECM1 heterozygous mice (ECM1-Het) and ECM1 hepatic parenchymatous cell-specific knockout mice (Alb-cre/ECM1 ^{Flox/flox}), because the ECM1 protein content in the liver extracellular matrix is lower, the progress rate of liver fibrosis is accelerated relative to ECM1 wild-type mice. However, when normal mice are modeled by liver fibrosis, because external pathogenic factors damage hepatic parenchymatous cell (Hepatocyte), the ability of hepatic parenchymatous cell (Hepatocyte) to produce ECM1 protein is weakened, and ECM1 protein in liver extracellular matrix is reduced, thus further promoting the activation of Latent TGF-β1 in liver and the progress of fibrosis.

ECM1 gene, as a gene is constitutively expressed in the liver, the expression is down-regulated during liver fibrosis, which promotes the development of liver fibrosis.

### References

1. Knock-in/Knock-out (KIKO) vectors for rapid integration of large DNA sequences, including whole metallic paths, into the Escherichia coli chromosome at well-characterized loci. Sabri S et al. Microb Cell Fact. (2013)
2. To knockout in 129 or in C57BL/6: That is the question. Seong E, Saunders TL, Stewart CL, Burmeister M. Trends Genet. 2004 Feb; 20 (2): 59-62.
3. Improved establishment of embryonic stem (ES) cell lines from the Chinese Kunming rice by hybridization with 129 rice. Yu S, Yan X, Liu H, Cai X, Cao S, Shen L, Zuo Z, Deng J, Ma X, Wang Y, Ren Z. Int J Mol Sci. 2014 Feb 25; 15 (3): 3389-402. doi: 10.3390/ijms15033389.
4. Gene transfer into cultured mammalian embryos by electroporation. Osumi N, Inoue T. Methods. 2001 May; 24 (1): 35-42.
5. Generation of Genetically Modified Mice through the Microinjection of Ocytes. Delerue F et al. J Vis Exp. (2017)
6. Pronuclear injection for the production of transgenic rice. Ittner LM et al. Nat Protoc. (2007)
7. Disease model discovery from 3,328 gene knockouts by The International Mouse PhenotypingConsortium. Meehan TF et al. Nat Genet. (2017)

## Claims

1. A preparation method of an animal model of non-human mammal liver fibrosis, comprising the following steps:
(a) providing a cell of a non-human mammal, inactivating the *ECM1* gene in the cell, thereby obtaining a non-human mammalian cell with inactivated *ECM1* gene; and
(b) using the cell with inactivated *ECM1* gene obtained in step (a), preparing an animal model of liver fibrosis with inactivated *ECM1* gene, wherein the animal model of liver fibrosis is an animal model of non-inducible spontaneous liver fibrosis, the non-human mammal is a mouse, the *ECM1* gene inactivation is systemic *ECM1* gene inactivation, and the animal model of non-human mammal liver fibrosis is homozygous and the animal model is a homozygous mouse with the age of 6-8 weeks;
wherein compared with the wild-type control animal, the animal model of the non-human mammal with inactivated *ECM1* gene obtained in the step (b) has one or more of the characteristics selected from the group consisting of:
(i) the increased progression of liver fibrosis;
(ii) the increased number of α-SMA positive cells;
(iii) the increased content of hydroxyproline;
(iv) the increased expression of genes related to liver fibrosis;
(v) the slightly increased content of aspartate aminotransferase (AST) and/or alanine aminotransferase (ALT);
(vi) the increased activation of the stellate cell HSC; and/or
(vii) the increased content of collagen.

2. The preparation method of claim 1, wherein the animal model of liver fibrosis is an animal model of early liver fibrosis.

## Patentansprüche

1. Herstellungsverfahren für ein Tiermodell für nichtmenschliche Säugetier- Leberfibrose, welches die folgenden Schritte umfasst:
(a) das Bereitstellen einer Zelle eines nichtmenschlichen Säugetiers, das Inaktivieren des ECM1-Gens in der Zelle, wodurch eine nichtmenschliche Säugetier-Zelle mit inaktiviertem ECM1-Gen erhalten wird; und
(b) das Verwenden der in Schritt (a) erhaltenen Zelle mit inaktiviertem ECM1-Gen, das Herstellen eines Tiermodells für Leberfibrose mit inaktiviertem ECM1-Gen, wobei das Tiermodell für Leberfibrose ein Tiermodell für nichtinduzierbare spontane Leberfibrose ist, wobei das nichtmenschliche Tier eine Maus ist, die ECM1-Gen-Inaktivierung systemische ECM1-Gen-Inaktivierung ist und das Tiermodell für nichtmenschliche Säugetier-Leberfibrose homozygot ist und das Tiermodell eine homozygote Maus im Alter von 6 bis 8 Wochen ist;
wobei im Vergleich zu einem Wildtyp-Kontrolltier das Tiermodell für das nichtmenschliche Säugetier mit inaktiviertem ECM1-Gen, das in Schritt (b) erhalten wird, ein oder mehrere Merkmale aufweist, die aus der aus den folgenden bestehenden Gruppe ausgewählt sind:
(i) verstärktes Fortschreiten von Leberfibrose;
(ii) erhöhte Anzahl an α-SMA-positiven Zellen;
(iii) erhöhter Gehalt an Hydroxyprolin;
(iv) erhöhte Expression von Genen im Zusammenhang mit Leberfibrose;
(v) leicht erhöhter Gehalt an Aspartat-Aminotransferase (AST) und/oder Alanin-Aminotransferase (ALT);
(vi) erhöhte Aktivierung der Sternzelle HSC; und/oder
(vii) erhöhter Gehalt an Collagen.

2. Herstellungsverfahren nach Anspruch 1, wobei das Tiermodell für Leberfibrose ein Tiermodell für frühe Leberfibrose ist.

## Revendications

1. Procédé de préparation d'un modèle animal de fibrose hépatique de mammifère non humain, comprenant les étapes suivantes consistant à :
(a) fournir une cellule d'un mammifère non humain, inactiver le gène *ECM1* dans la cellule, en obtenant ainsi une cellule de mammifère non humain avec le gène *ECM1* inactivé ; et
(b) en utilisant la cellule avec le gène *ECM1* inactivé obtenue à l'étape (a), préparer un modèle animal de fibrose hépatique avec le gène *ECM1* inactivé, dans lequel le modèle animal de fibrose hépatique est un modèle animal de fibrose hépatique spontanée non inductible, le mammifère non humain est une souris, l'inactivation du gène *ECM1* est une inactivation systémique du gène *ECM1,* et le modèle animal de fibrose hépatique de mammifère non humain est homozygote et le modèle animal est une souris homozygote âgée de 6 à8 semaines ;
dans lequel, par rapport à l'animal témoin de type sauvage, le modèle animal du mammifère non humain avec le gène *ECM1* inactivé obtenu à l'étape (b) présente une ou plusieurs des caractéristiques sélectionnées dans le groupe comprenant :
(i) la progression accrue de la fibrose hépatique ;
(ii) le nombre accru de cellules a-SMA positives ;
(iii) la teneur accrue en hydroxyproline ;
(iv) l'expression accrue de gènes liés à la fibrose hépatique ;
(v) la teneur légèrement accrue en aspartate aminotransférase (ASAT) et/ou en alanine aminotransférase (ALAT) ;
(vi) l'activation accrue des cellules stellaires hépatiques CSR ; et/ou
(vii) la teneur accrue en collagène.

2. Procédé de préparation selon la revendication 1, dans lequel le modèle animal de fibrose hépatique est un modèle animal de fibrose hépatique précoce.
